# EUROPÄISCHE PATENTSCHRIFT

(11) **EP 1 444 992 B1**
(45) Veröffentlichungstag und Bekanntmachung des Hinweises auf die Patenterteilung: **21.12.2005**
(21) Anmeldenummer: 03002869.0
(22) Anmeldetag: 08.02.2003
(51) Int. Cl.: A61L 2/24, A61L 2/26

(54) **Verfahren zum Reinigen, Desinfizieren und Sterilisieren von Instrumenten**
Process for cleaning, disinfecting and sterilizing instruments
Procédé de nettoyage, désinfection et stérilisation des instruments

(43) Veröffentlichungstag der Anmeldung: 11.08.2004
(73) Patentinhaber: VSE Aktiengesellschaft, 66121 Saarbrücken (DE)
(72) Erfinder: Schrickel, Ralph, Dipl.-Ing., 66123 Saarbrücken (DE)
(74) Vertreter: Rohmann, Michael

(56) Entgegenhaltungen:
- DE-A- 10 019 113
- DE-A- 19 514 284
- US-A- 5 374 813

## Beschreibung

Die Erfindung betrifft ein Verfahren zum Reinigen, Desinfizieren und Sterilisieren von Sterilinstrumenten. - Sterilinstrumente meint Gegenstände, insbesondere aus der Medizintechnik, die steril bzw. sterilisiert zum Einsatz gelangen und nach ihrer bestimmungsgemäßen Verwendung wieder gereinigt, desinfiziert und sterilisiert werden müssen. Sterilinstrumente meint vor allem chirurgische Instrumente, sterile Verbrauchs- und Hilfsmittel sowie wiederverwendbare sterile Medizinprodukte. Mit dem Begriff Sterilinstrumente sind im Rahmen der Erfindung auch thermolabile Güter sowie sterilisierbare Wäsche gemeint.

Die aus der Praxis bekannten Verfahren der eingangs genannten Art arbeiten in der Regel mit einer Mehrzahl aufwendiger und zeitintensiver Verfahrensschritte. Zwischen den einzelnen Verfahrensschritten müssen oftmals längere Pausen bzw. Stillstandszeiten eingehalten werden. Vor allem die korrekte Zuordnung der Sterilinstrumente zum Herkunftsort und auch die richtige Zuordnung zu den in Frage kommenden Reinigungs-, Desinfektions- und insbesondere Sterilisierungsmaßnahmen ist oftmals problematisch, aufwendig und fehleranfällig. Das kann im Extremfall dazu führen, dass Sterilinstrumente nicht korrekt bzw. unzureichend behandelt werden und vor allem nicht ausreichend sterilisiert sind.

DE-A-19514284 offenbart ein System zur Dokumentation des Reinigungsprozesses von Operationsinstrumenten, das die Reinigungsprozessdaten für jede Reinigungs-Untereinheit eines Instrumentensets individuell dokumentiert und die Zuordnung der Instrumente und der damit verknüpften Daten nachweisbar macht.

Demgegenüber liegt der Erfindung das technische Problem zugrunde, ein Verfahren der eingangs genannten Art anzugeben, mit dem die vorstehend geschilderten Nachteile effektiv vermieden werden können.

Zur Lösung dieses technischen Problems lehrt die Erfindung ein Verfahren zum Reinigen, Desinfizieren und Sterilisieren von Sterilinstrumenten,
wobei Aufnahmebehälter, die jeweils eine Mehrzahl von Sterilinstrumenten enthalten, in Chargenwagen angeliefert werden und wobei zumindest jeder Chargenwagen EDV-mäßig erfasst wird,
wobei die Aufnahmebehälter dem Chargenwagen und die einzelnen Sterilinstrumente dem zugeordneten Aufnahmebehälter entnommen werden und wobei die Sterilinstrumente und der Aufnahmebehälter in einer Reinigungs- und Desinfektionsvorrichtung gereinigt und desinfiziert werden,
wobei anschließend die gereinigten und desinfizierten Sterilinstrumente nach einem vorgegebenen Muster in den zugeordneten Aufnahmebehälter gepackt werden und der Aufnahmebehälter daraufhin in den Chargenwagen überführt wird,
wobei der Chargenwagen im Anschluss daran mit den geschlossenen Aufnahmebehältern in einer Sterilisationseinrichtung sterilisiert wird
und wobei die sterilisierten Chargenwagen mit den in den Aufnahmebehältern aufgenommenen Sterilelementen ihrem Verwendungsort zugeführt werden. Dass die Sterilinstrumente in den Aufnahmebehälter gepackt werden meint im Rahmen der Erfindung insbesondere, dass die Sterilinstrumente auf einen Siebeinsatz des Aufnahmebehälters gepackt werden.

Benutzte Sterilinstrumente werden also beispielsweise aus einem Krankenhaus (Herkunftsort) angeliefert und mit anderen Sterilinstrumenten, die beispielsweise aus anderen Krankenhäusern stammen, in der ZRDS zentral gereinigt, desinfiziert und sterilisiert. ZRDS steht hier und nachfolgend für eine erfindungsgemäße zentrale Reinigungs-, Desinfektions- und Sterilisationsanlage für die Durchführung des erfindungsgemäßen Verfahrens. Die Sterilinstrumente werden in der Regel mit einem Transportfahrzeug von ihrem Herkunftsort angeliefert, wobei es sich beispielsweise um einen Kleintransporter handelt. Die Transportfahrzeuge nehmen ihrerseits Chargenwagen auf, in denen jeweils eine Charge von Sterilinstrumenten enthalten ist. Es liegt im Rahmen der Erfindung, dass zumindest ein Chargenwagen von einem geschlossenen Transportwagen aufgenommen wird, welcher Transportwagen wiederum von dem Transportfahrzeug, z. B. einem Kleintransporter, aufgenommen wird. Beispielsweise wird ein einziger Chargenwagen in einem Transportwagen aufgenommen. Der Transportwagen bildet für diesen Chargenwagen gleichsam eine zweite Hülle bzw. eine Umverpackung oder Schutzverpackung. Ein Chargenwagen hat nach einer Ausführungsform der Erfindung keine unterseitigen Räder bzw. Rollen und ist deshalb nicht selbständig verfahrbar. Deshalb wird der Chargenwagen in der ZRDS zweckmäßigerweise auf einem verfahrbaren Unterwagen aufgenommen, mit dem der Chargenwagen dann zumindest durch einen Teil der ZRDS verfahren werden kann.

Nach sehr bevorzugter Ausführungsform der Erfindung weist jeder Chargenwagen für die EDV-mäßige Erfassung zumindest einen Informationsträger auf, von welchem Informationsträger Daten in einen Computer bzw. in eine EDV-Anlage eingelesen werden, welche Daten bei der weiteren Behandlung des Chargenwagens bzw. des Inhaltes des Chargenwagens verwertet werden. Bei dem Informationsträger handelt es sich vorzugsweise um einen Barcode bzw. um ein Etikett mit einem Barcode. Der Informationsträger vermittelt zweckmäßigerweise Informationen über die Herkunft und/oder über die Art der Sterilinstrumente und/oder über die Behandlungsparameter der Sterilinstrumente. Vorzugsweise ist jeder Aufnahmebehälter eines Chargenwagens mit einem solchen Informationsträger ausgerüstet. In einem Chargenwagen wird in der Regel eine Mehrzahl von Aufnahmebehältern für die Sterilinstrumente aufgenommen. Ein Aufnahmebehälter besteht vorzugsweise aus einem Behälterdeckel, einem Behälterunterteil und einem in dem Aufnahmebehälter aufnehmbaren Siebeinsatz, auf dem die Sterilinstrumente angeordnet sind.

Nach bevorzugter Ausführungsform der Erfindung werden der Aufnahmebehälter und die Sterilinstrumente von einem ersten Raum aus in eine dem ersten Raum zugewandte Öffnung der Reinigungs- und Desinfektionsvorrichtung eingeführt und die gereinigten und desinfizierten Sterilinstrumente sowie der Aufnahmebehälter werden in einem zweiten Raum aus einer dem zweiten Raum zugewandten Öffnung der Reinigungs- und Desinfektionsvorrichtung entnommen. Es liegt dabei im Rahmen der Erfindung, dass ein Aufnahmebehälter in seine Bestandteile, nämlich den Behälterdeckel, das Behälterunterteil und den Siebeinsatz aufgetrennt wird und diese Bestandteile einzeln in der Reinigungs- und Desinfektionsvorrichtung gereinigt und desinfiziert werden. Zweckmäßigerweise werden auch die Sterilinstrumente von dem zugeordneten Siebeinsatz genommen und in die Reinigungs- und Desinfektionsvorrichtung eingeführt. Die erfindungsgemäße Reinigungs- und Desinfektionsvorrichtung bildet gleichsam eine Schleuse zwischen dem ersten Raum und dem zweiten Raum. In dem ersten Raum, dem sogenannten "unreinen Raum" werden zweckmäßigerweise Behälterdeckel, Behälterunterteil und Siebeinsatz der Aufnahmebehälter voneinander getrennt und einzeln in die Reinigungs- und Desinfektionsvorrichtung eingeführt. Die Reinigung kann insbesondere mit enzymatischen Reinigungsmitteln erfolgen, die biologisch leicht abzubauen sind. Nach einer bevorzugten Ausführungsform erfolgt die Reinigung mit Hilfe von Ultraschall. Auf diese Weise können Verkrustungen und Anbackungen effektiv von den Komponenten entfernt werden. Die Desinfektion der Sterilinstrumente erfolgt zweckmäßigerweise thermisch, d. h. durch Erhitzen. Die Desinfektionszeit wird vorzugsweise entsprechend der Vorgabe der Richtlinie des Robert-Koch-Institutes eingehalten. Der zweite Raum, der sogenannte "reine Raum" dient als Packraum. Hier erfolgt ein erneutes Packen der Siebeinsätze. Es wird jeweils eine Mehrzahl der gereinigten und desinfizierten Sterilinstrumente nach einem vorgegebenen Muster auf einen zugeordneten Siebeinsatz gepackt und der Siebeinsatz mit den darauf aufgenommenen Sterilinstrumenten wird dann in einen Aufnahmebehälter überführt. - Parallel zur Reinigung und Desinfektion der Sterilinstrumente und der Aufnahmebehälter wird vorzugsweise auch der Chargenwagen gereinigt und desinfiziert und bevorzugt wird auch der Transportwagen zumindest desinfiziert.

Nach sehr bevorzugter Ausführungsform, der im Rahmen der Erfindung ganz besondere Bedeutung zukommt, werden die Sterilinstrumente nach einem in der EDV-Anlage gespeicherten Packmuster in den jeweils zugeordneten Aufnahmebehälter gepackt. Es liegt dabei im Rahmen der Erfindung, dass die Sterilelemente nach dem genannten Packmuster auf einen zugeordneten Siebeinsatz des Aufnahmebehälters gepackt werden. Für jeden Siebeinsatz bzw. für jeden Aufnahmebehälter ist also zweckmäßigerweise ein Packmuster bzw. sind die dem Packmuster entsprechenden Daten in der EDV-Anlage abgespeichert. Nach einer Ausführungsform kann das Packmuster bzw. die entsprechenden Daten bei dem anfänglichen EDV-mäßigen Erfassen des Chargenwagens bzw. seiner Komponenten eingelesen werden. Vorzugsweise ist für jeden Siebeinsatz bzw. für jeden Aufnahmebehälter ein Bild des fertig gepackten Siebeinsatzes bzw. Aufnahmebehälters in der EDV-Anlage abgespeichert, so dass eine einfache optische Kontrolle über einen Bildschirm der EDV-Anlage möglich ist.

Nach dem Packen der Siebeinsätze bzw. der Aufnahmebehälter wird für jeden Aufnahmebehälter ein Behälterinformationsträger erstellt, zweckmäßigerweise ein entsprechendes Etikett gedruckt, auf dem die Daten für die Komponenten des Aufnahmebehälters aufgenommen sind. Ein gedrucktes Etikett kann beispielsweise in ein entsprechendes Fenster eines Aufnahmebehälters eingeschoben werden. Der Behälterinformationsträger bzw. das auf dem Aufnahmebehälter vorhandene Etikett vermittelt insbesondere Informationen bezüglich des Packdatums und des Verfallsdatums. Weiterhin können auch Informationen zur Etikettennummer, Siebeinsatznummer, Siebeinsatzbezeichnung oder Packernummer enthalten bzw. aufgedruckt sein. Außerdem können von dem Behälterinformationsträger, insbesondere von dem Etikett Informationen bzw. Daten für die spätere Sterilisation vermittelt werden.

Es liegt im Rahmen der Erfindung, dass die Sterilisation durch Erhitzen der Sterilinstrumente bzw. der Aufnahmebehälter mit den Sterilinstrumenten erfolgt. Nach sehr bevorzugter Ausführungsform der Erfindung werden auf einen Siebeinsatz und in einen zugeordneten Aufnahmebehälter jeweils nur Sterilinstrumente gepackt, die bei gleichen Bedingungen, insbesondere bei gleicher Sterilisiertemperatur sterilisiert werden. Es liegt weiterhin im Rahmen der Erfindung, dass vor der Sterilisation in einen Chargenwagen nur Sterilinstrumente (bzw. nur Siebeinsätze und Aufnahmebehälter) gepackt werden, die bei gleichen Sterilisierbedingungen, insbesondere bei gleicher Sterilisiertemperatur sterilisiert werden.

Nach bevorzugter Ausführungsform der Erfindung werden vor der Sterilisation Chargeninformationsträger erstellt und jeder Chargenwagen wird mit einem zugeordneten Chargeninformationsträger versehen, von welchem Chargeninformationsträger unter anderem Daten für die nachfolgende Sterilisation von der EDV-Anlage eingelesen werden. Vorzugsweise wird als Chargeninformationsträger ein Chargenetikett gedruckt, das zweckmäßigerweise alle Daten der Behälterinformationsträger bzw. der oben beschriebenen Behälteretiketten beinhaltet. - Nach sehr bevorzugter Ausführungsform wird bereits vor der Erstellung des Chargeninformationsträgers mit Hilfe eines Chargendatenverarbeitungsprogrammes die Zusammenstellung von Aufnahmebehältern bzw. Siebeinsätzen zu einer Charge verweigert, wenn dadurch Sterilinstrumente zusammengestellt würden, die bei unterschiedlichen Sterilisierbedingungen, insbesondere bei unterschiedlicher Sterilisiertemperatur sterilisiert werden müssten.

Vorzugsweise werden vor der Sterilisationseinrichtung bzw. an der Sterilisationseinrichtung die Daten einer Mehrzahl von Chargeninformationsträgern von der EDV-Anlage gleichzeitig eingelesen und es wird zunächst die Charge bzw. die Chargen mit der niedrigsten Sterilisationstemperatur ausgewählt und anschließend in der Sterilisationseinrichtung sterilisiert. Charge meint hier einen Chargenwagen. -Die EDV-Anlage der ZRDS kann beispielsweise aus mehreren vernetzten Computern bestehen. Zumindest ein Computer bzw. Mikroprozessor ist der Sterilisatinseinrichtung zugeordnet.

Es liegt im Rahmen der Erfindung, dass in der Sterilisationseinrichtung mit Öffnungen versehene Chargenwagen und darin aufgenommene verschlossene Aufnahmebehälter sterilisiert werden. Mit Öffnungen versehener Chargenwagen meint insbesondere einen Chargenwagen mit gitterartigen Wänden. Die Aufnahmebehälter sind zweckmäßigerweise fest verschlossen und verplombt. Nach sehr bevorzugter Ausführungsform ist in der Wandung eines Aufnahmebehälters aber zumindest ein Filter vorgesehen, der den bei der Sterilisation entstehenden Dampf nach außen entweichen lässt. Nach diesem Entweichen des Dampfes schließen sich vorzugsweise die Poren des Filters. - Es liegt im Rahmen der Erfindung, dass eine thermische Sterilisation durchgeführt wird. Vorzugsweise findet eine Plasmasterilisation der Sterilinstrumente statt.

Es liegt im Rahmen der Erfindung, dass nach der Sterilisation ein Abkühlen des heißen Chargenwagens stattfindet.-Zuletzt erfolgt das "Ausbuchen" eines Chargenwagens. Mit anderen Worten wird EDV-mäßig erfasst, dass ein bestimmter Chargenwagen die ZRDS verlässt. Ein solcher Chargenwagen wird zweckmäßigerweise wieder in einen verschließbaren bzw. geschlossenen Transportwagen überführt, und dieser Transportwagen wird in ein Transportfahrzeug, zum Beispiel einen Kleintransporter eingebracht und zum Bestimmungsort, beispielsweise zu einem Krankenhaus transportiert.

Der Erfindung liegt die Erkenntnis zugrunde, dass mit dem erfindungsgemäßen Verfahren eine einfache, wenig aufwendige und zügige Reinigung, Desinfektion und Sterilisation von Sterilinstrumenten möglich ist. Die Verfahrensschritte bzw. die entsprechenden Behandlungsvorrichtungen sind so miteinander verbunden bzw. kombiniert, dass störende Pausen oder längere Stillstandszeiten vermieden werden können. Von besonderer Bedeutung ist im Rahmen der Erfindung, dass eine einwandfreie und korrekte Zuordnung der Sterilinstrumente zum Herkunftsort bzw. zum Bestimmungsziel und insbesondere zu den für diese Sterilinstrumente in Frage kommenden Behandlungsmaßnahmen möglich ist. Im Ergebnis wird eine optimale Reinigung, Desinfektion und Sterilisation der Sterilinstrumente erzielt.

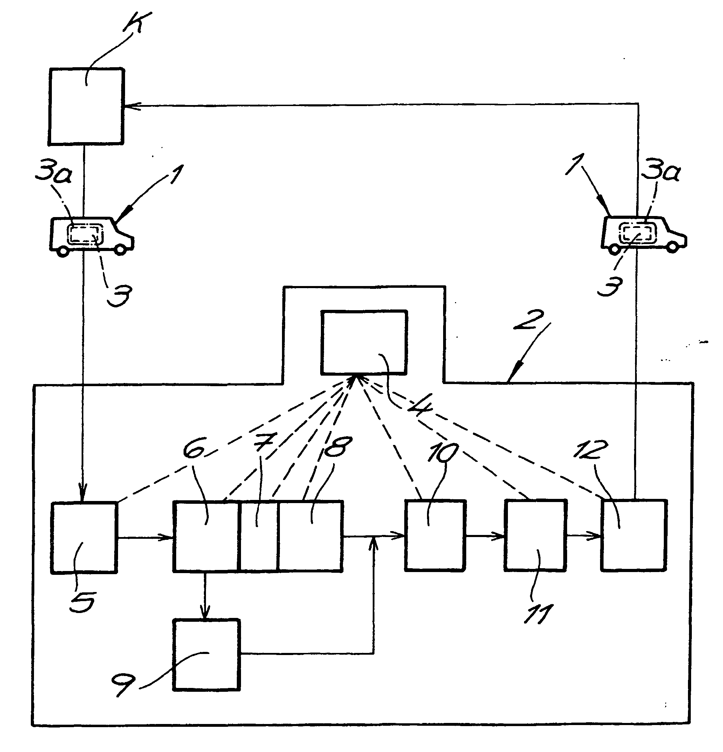

Nachfolgend wird die Erfindung anhand einer lediglich ein Ausführungsbeispiel darstellenden Zeichnung näher erläutert. Die einzige Figur zeigt ein Schema für die Abfolge der Verfahrensschritte des erfindungsgemäßen Verfahrens.

Benutzte und somit zu reinigende, zu desinfizierende und zu sterilisierende Sterilinstrumente werden von einem Krankenhaus K mit einem Transportfahrzeug 1 zur zentralen Reinigungs-, Desinfektions- und Sterilisationsanlage 2 transportiert. Die Sterilinstrumente befinden sich dabei in einem Chargenwagen 3, der in der Figur bzw. in dem Transportfahrzeug 1 gestrichelt angedeutet wurde. Der Chargenwagen 3 wird wiederum von einem geschlossenen Transportwagen 3a aufgenommen, welcher Transportwagen 3a in der Figur bzw. in dem Transportfahrzeug 1 strichpunktiert angedeutet wurde. Sobald das Transportfahrzeug 1 die ZRDS 2 erreicht hat, findet zunächst ein "Einbuchen" des Transportwagens 3a bzw. des Chargenwagens 3 in der Einbuchstation 5 statt. Mit anderen Worten wird der Chargenwagen 3 bzw. die zugehörige Charge EDV-mäßig erfasst. Die gestrichelten Linien verdeutlichen den Anschluss der Einbuchstation 5 bzw. der anderen Komponenten/Stationen an eine EDV-Anlage 4. Hierzu weist der Chargenwagen 3 zumindest einen Informationsträger für die EDV-mäßige Erfassung durch die EDV-Anlage 4 auf. Von diesem Informationsträger können Daten eingelesen werden, die bei der weiteren Behandlung des Chargenwagens 3 bzw. des Inhaltes des Chargenwagens 3 verwertet werden. Der Informationsträger kann in Form eines Barcodes bzw. in Form eines Etiketts mit einem Barcode an dem Chargenwagen 3 vorgesehen sein.

In einem ersten "unreinen" Raum 6 werden die nicht dargestellten Aufnahmebehälter mit den darin aufgenommenen Sterilinstrumenten dem Chargenwagen 3 entnommen. Anschließend wird jeder Aufnahmebehälter in Behälterdeckel, Behälterunterteil und Siebeinsatz getrennt und die auf dem Siebeinsatz aufgenommenen Sterilinstrumente werden von dem Siebeinsatz genommen. Alle vorgenannten Komponenten werden dann von dem ersten "unreinen" Raum 6 aus in eine dem ersten Raum 6 zugewandte Öffnung der Reinigungs- und Desinfektionsvorrichtung 7 eingeführt. Alle Komponenten werden hier gereinigt und desinfiziert. Die gereinigten und desinfizierten Komponenten (Behälterdeckel, Behälterunterteil, Siebeinsatz und Sterilinstrumente) werden in einem zweiten "reinen" Raum 8 einer diesem zweiten Raum 8 zugewandten Öffnung der Reinigungs- und Desinfektionsvorrichtung 7 entnommen. Die Reinigungs- und Desinfektionsvorrichtung 7 bildet also gleichsam eine Schleuse zwischen dem ersten "unreinen" Raum 6 und dem zweiten "reinen" Raum 8. Parallel wird im Ausführungsbeispiel der Chargenwagen 3 in einer Einrichtung 9 gereinigt und desinfiziert.

Der zweite "reine" Raum 8 dient als Packraum, in dem die gereinigten und desinfizierten Sterilinstrumente erneut nach einem vorgegebenen Muster auf einen zugeordneten Siebeinsatz gepackt werden. Dabei werden die Sterilinstrumente nach einem in der EDV-Anlage 4 gespeicherten Packmuster auf den zugeordneten Siebeinsatz gepackt. Zu jedem Siebeinsatz ist vorzugsweise ein Bild des fertig gepackten Siebeinsatzes in der EDV-Anlage abgespeichert und auf einem Bildschirm abrufbar. Hierdurch wird eine einfache optische Kontrolle des Packens der Siebeinsätze gewährleistet. Anschließend werden die gepackten Siebeinsätze wieder in die Aufnahmebehälter eingesetzt. Es liegt dabei im Rahmen der Erfindung, dass auf einen Siebeinsatz und in einen Aufnahmebehälter jeweils nur Sterilinstrumente gepackt werden, die bei gleichen Bedingungen, insbesondere bei gleicher Sterilisiertemperatur nachfolgend sterilisiert werden. In der Beschickstation bzw. Beladestation 10 werden die Aufnahmebehälter wieder in die Chargenwagen 3 eingebracht. Auch hier gilt, dass in einen Chargenwagen 3 nur Sterilinstrumente bzw. Aufnahmebehälter gepackt werden, die bei gleichen Sterilisierbedingungen, insbesondere bei gleicher Sterilisiertemperatur sterilisiert werden. Dabei wird zweckmäßigerweise jeder Chargenwagen 3 mit einem zugeordneten Chargeninformationsträger, vorzugsweise mit einem Chargenetikett versehen, von welchem Chargeninformationsträger Daten für die nachfolgende Sterilisation in die EDV-Anlage 4 eingelesen werden können. Anschließend werden die Chargenwagen 3 zur Sterilisation in die Sterilisationseinrichtung 11 überführt. Die Sterilisation erfolgt hier thermisch, d. h. durch Erhitzen. Im Anschluss an die Sterilisation werden die heißen Chargenwagen 3 abgekühlt. Daraufhin erfolgt ein EDV-mäßiges "Ausbuchen" der Chargenwagen 3 in der Ausbuchstation 12. Die Chargenwagen 3 werden dann wieder in Transportwagen 3a eingebracht, welche Transportwagen 3a mit einem Transportfahrzeug 1 zu ihrem Bestimmungsort, im Ausführungsbeispiel zum Krankenhaus K befördert werden.

## Patentansprüche

1. Verfahren zum Reinigen, Desinfizieren und Sterilisieren von Sterilinstrumenten,
wobei Aufnahmebehälter, die jeweils eine Mehrzahl von Sterilinstrumenten enthalten, in Chargenwagen (3) angeliefert werden und wobei zumindest jeder Chargenwagen (3) EDV-mäßig erfasst wird,
wobei die Aufnahmebehälter dem Chargenwagen (3) und die Sterilinstrumente dem zugeordneten Aufnahmebehälter entnommen werden und wobei die Sterilinstrumente und der Aufnahmebehälter in einer Reinigungs- und Desinfektionsvorrichtung (7) gereinigt und desinfiziert werden,
wobei anschließend die gereinigten und desinfizierten Sterilinstrumente nach einem vorgegebenen Muster in den zugeordneten Aufnahmebehälter gepackt werden und der Aufnahmebehälter daraufhin in den Chargenwagen überführt wird,
wobei der Chargenwagen (3) im Anschluss daran mit den Aufnahmebehältern in einer Sterilisationseinrichtung (11) sterilisiert wird
und wobei die sterilisierten Chargenwagen (3) mit den in den Aufnahmebehältern aufgenommenen Sterilelementen ihrem Verwendungsort zugeführt werden.

2. Verfahren nach Anspruch 1, wobei jeder Chargenwagen (3) für die EDV-mäßige Erfassung zumindest einen Informationsträger aufweist, von welchem Informationsträger Daten in einen Computer bzw. in eine EDV-Anlage (4) eingelesen werden, welche Daten bei der weiteren Behandlung des Chargenwagens (3) bzw. des Inhaltes des Chargenwagens (3) verwertet werden.

3. Verfahren nach einem der Ansprüche 1 oder 2, wobei die Sterilinstrumente und der Aufnahmebehälter von einem ersten Raum (6) aus in eine dem ersten Raum (6) zugewandte Öffnung der Reinigungs- und Desinfektionsvorrichtung (7) eingeführt werden und wobei die gereinigten und desinfizierten Sterilinstrumente und Aufnahmebehälter in einem zweiten Raum (8) aus einer dem zweiten Raum (8) zugewandten Öffnung der Reinigungs- und Desinfektionsvorrichtung (7) entnommen werden.

4. Verfahren nach einem der Ansprüche 1 bis 3, wobei die Sterilinstrumente nach einem in der EDV-Anlage (4) gespeicherten Packmuster in den jeweils zugeordneten Aufnahmebehälter gepackt werden.

5. Verfahren nach einem der Ansprüche 1 bis 4, wobei auf einen Siebeinsatz und in einen Aufnahmebehälter jeweils nur Sterilinstrumente gepackt werden, die bei gleichen Bedingungen, insbesondere bei gleicher Sterilisiertemperatur sterilisiert werden.

6. Verfahren nach Anspruch 5, wobei vor der Sterilisation in einen Chargenwagen (3) nur Sterilinstrumente gepackt werden, die bei gleichen Sterilisierbedingungen, insbesondere bei gleicher Sterilisiertemperatur sterilisiert werden.

7. Verfahren nach einem der Ansprüche 1 bis 6, wobei Chargeninformationsträger erstellt werden und jeder Chargenwagen (3) mit einem zugeordneten Chargeninformationsträger versehen wird, von welchem Chargeninformationsträger unter anderem Daten für die nachfolgende Sterilisation von der EDV-Anlage (4) eingelesen werden.

8. Verfahren nach Anspruch 7, wobei bereits vor Erstellung des Chargeninformationsträgers mit Hilfe eines Chargenprogrammes die Zusammenstellung von Aufnahmebehältern bzw. Siebeinsätzen zu einer Charge verweigert wird, wenn **dadurch** Sterilinstrumente zusammengestellt würden, die bei unterschiedlichen Sterilisierbedingungen, insbesondere bei unterschiedlicher Sterilisiertemperatur sterilisiert werden müssten.

9. Verfahren nach einem der Ansprüche 7 oder 8, wobei vor der Sterilisationseinrichtung (11) bzw. an der Sterilisationseinrichtung (11) die Daten einer Mehrzahl von Chargeninformationsträgern von der EDV-Anlage eingelesen werden können und wobei zunächst die Charge bzw. die Chargen mit der niedrigsten Sterilisiertemperatur ausgewählt werden und anschließend in der Sterilisationseinrichtung (11) sterilisiert werden.

10. Verfahren nach einem der Ansprüche 1 bis 9, wobei in der Sterilisationseinrichtung mit Öffnungen versehene Chargenwagen (3) und darin aufgenommene verschlossene Aufnahmebehälter sterilisiert werden.

## Claims

1. Process for the cleaning, disinfecting and sterilisation of sterile instruments,
whereby receptacles, which respectively contain a number of sterile instruments, are delivered in charge carriages (3) and whereby at least each charge carriage (3) is accounted for by means of EDP,
in which the receptacles are taken from the charge carriage (3) and the sterile instruments are taken from the allocated receptacle and in which the sterile instruments and the receptacle are cleaned and disinfected in a cleaning and disinfecting device (7),
whereby the cleaned and disinfected sterile instruments are then packed in the allocated receptacle in accordance with a prescribed pattern and the receptacle is then transferred to the charge carriage,
whereby the charge carriage (3) and the receptacles are then sterilised in a sterilisation device (11),
and whereby the sterilised charge carriages (3) with the sterile elements held in the receptacles are transported to their point of use.

2. Process in accordance with Claim 1, in which each charge carriage (3) has at least one information carrier for the acquisition of data by EDP means and from which data can be read into a computer or into an EDP system (4), which data is evaluated in the subsequent handling of the charge carriage (3) and of the contents of the charge carriage (3).

3. Process in accordance with either of the Claims 1 or 2, whereby the sterile instruments and the receptacle are taken from a first room (6) in through an opening of the cleaning and disinfection device facing the first room (6) and whereby the cleaned and disinfected sterile instruments and receptacle are taken to a second room (8) through an opening in the cleaning and disinfecting device (7) facing the second room (8).

4. Process in accordance with any of the Claims 1 to 3, whereby the sterile instruments are packed in the respective allocated receptacle in accordance with a packaging pattern stored in the EDP system (4).

5. Process in accordance with any of the Claims 1 to 4, whereby only sterile instruments which have been sterilised under the same conditions, in particular at the same sterilisation temperature, are respectively packed on a mesh insert and in a receptacle.

6. Process in accordance with Claim 5, in which before the sterilisation only sterile instruments, which have been sterilised under the same conditions, in particular at the same sterilisation temperature, are packed in a charge carriage (3).

7. Process in accordance with any of the Claims 1 to 6, whereby charge information carriers are prepared and each charge carriage (3) is provided with an allocated charge information carrier from which, together with other data, data for the subsequent sterilisation is read in by the EDP system (4).

8. Process in accordance with Claim 7, whereby before the preparation of the charge information carrier using a charge program, the assembly of receptacles and mesh inserts for a charge is rejected when sterile instruments are thus assembled which had to be sterilised under different sterilisation conditions, and in particular at different sterilisation temperatures.

9. Process in accordance with one of the Claims 7 or 8, whereby ahead of the sterilisation device (11) or at the sterilisation device (11) the data from a number of charge information carriers can be read in by the EDP system and whereby the charge or the charges with the lowest sterilisation temperature are first selected and then sterilised in the sterilisation device (11).

10. Process in accordance with any of the Claims 1 to 9, whereby charge carriers (3) provided with openings and the closed receptacles held therin are sterilised in the sterilisation device.

## Revendications

1. Procédé pour nettoyer, désinfecter et stériliser des instruments devant être stériles,
dans lequel des récipients de recueil qui contiennent chacun une pluralité d'instruments devant être stériles sont chargés dans des véhicules (3) et dans lequel au moins chacun des véhicules (3) est identifié en fonction de données saisies par un dispositif de traitement d'informations,
dans lequel les récipients de recueil sont retirés du véhicule (3) et les instruments à stériliser sont retirés de leur récipient associé et les instruments à stériliser ainsi que les récipients de recueil sont nettoyés et désinfectés dans un dispositif de nettoyage et de désinfection (7),
dans lequel les instruments à stériliser nettoyés et désinfectés sont ensuite disposés selon un schéma prédéterminé dans les récipients de recueil associés, à la suite de quoi les récipients de recueil sont transférés dans le véhicule,
dans lequel le véhicule (3) contenant les récipients de recueil est par la suite stérilisé dans un dispositif de stérilisation (11),
et dans lequel les véhicules (3) stérilisés contenant les éléments stériles disposés dans les récipients de recueil sont conduits jusqu'au lieu d'utilisation de ceux-ci.

2. Procédé selon la revendication 1, dans lequel chaque véhicule (3) présente au moins un support d'informations pour la saisie de données effectuée par le dispositif de traitement d'informations, lequel support d'informations permet à des données d'être lues par un ordinateur ou par une installation de traitement d'informations (4), lesquelles données sont exploitées lors du traitement subséquent du véhicule (3) ou du contenu de ce véhicule (3).

3. Procédé selon l'une des revendications 1 ou 2, dans lequel les instruments à stériliser et le récipient de recueil sont transférés depuis une première chambre (6) dans une ouverture du dispositif de nettoyage et de désinfection (7) communiquant avec cette première chambre (6), et dans lequel les instruments et les récipients de recueil nettoyés et désinfectés se retirent dans une seconde chambre (8) par une ouverture du dispositif de nettoyage et de désinfection (7) communiquant avec cette seconde chambre (8).

4. Procédé selon l'une des revendications 1 à 3, dans lequel les instruments à stériliser sont conditionnés dans chacun des récipients de recueil associés suivant un schéma de conditionnement enregistré dans la mémoire de l'installation de traitement d'informations (4).

5. Procédé selon l'une des revendications 1 à 4, dans lequel on ne conditionne respectivement sur une grille et dans un récipient de recueil que des instruments devant être stérilisés dans des conditions similaires, notamment à une température de stérilisation similaire.

6. Procédé selon la revendication 5, dans lequel on ne conditionne dans un véhicule (3), préalablement à la stérilisation, que des instruments devant être stérilisés dans des conditions similaires, notamment à une température de stérilisation similaire.

7. Procédé selon l'une des revendications 1 à 6, dans lequel des supports d'informations relatifs à la charge du véhicule sont établis et chaque véhicule (3) est muni d'un tel support d'informations qui lui est associé, lequel support d'informations permet à une installation de traitement d'informations (4) de lire des données notamment relatives à la stérilisation à effectuer subséquemment.

8. Procédé selon la revendication 7, dans lequel le rassemblement des récipients de recueil ou des grilles en une charge est refusé au moyen d'un programme de charge bien avant l'établissement du support d'informations relatif à la charge du véhicule lorsqu'il aurait été rassemblé des instruments qui auraient dû être stérilisés dans des conditions de stérilisation différentes, notamment à une température de stérilisation différente.

9. Procédé selon l'une des revendications 7 ou 8, dans lequel les données d'une pturatité de supports d'informations relatifs à la charge peuvent être lues par une installation de traitement d'informations avant le dispositif de stérilisation (11) ou au niveau du dispositif de stérilisation (11) et dans lequel sont tout d'abord choisies la charge ou les charges à température de stérilisation la plus basse qui sont ensuite stérilisées dans le dispositif de stérilisation (11).

10. Procédé selon l'une des revendications 1 à 9, dans lequel on stérilise dans le dispositif de stérilisation des véhicules (3) pourvus d'ouvertures ainsi que des récipients de recueil fermés contenus dans ceux-ci.
